(19) Europäisches Patentamt
European Patent Office
Office européen des brevets

(11) Veröffentlichungsnummer: **0 130 281**
**A1**

(12) # EUROPÄISCHE PATENTANMELDUNG

(21) Anmeldenummer: **84103508.2**

(51) Int. Cl.⁴: **A 23 K 1/18**, A 23 K 1/16

(22) Anmeldetag: **30.03.84**

(30) Priorität: **30.06.83 DE 3323508**

(43) Veröffentlichungstag der Anmeldung: **09.01.85 Patentblatt 85/2**

(84) Benannte Vertragsstaaten: **AT BE CH DE FR GB IT LI LU NL SE**

(71) Anmelder: **Degussa Aktiengesellschaft, Weissfrauenstrasse 9, D-6000 Frankfurt am Main 1 (DE)**

(72) Erfinder: **Koch, Friedhelm, Dr. Dipl.-Chem., Glatzerstrasse 22, D-6451 Grosskrotzenburg (DE)**
Erfinder: **Spindler, Manfred, Dr. Dipl.-Chem., Kurfürstenstrasse 24, D-6450 Hanau 1 (DE)**
Erfinder: **Tanner, Herbert, Dr. Dipl.-Chem., Wildaustrasse 20, D-6450 Hanau 9 (DE)**

(54) **Verwendung von Salzen des Methionins zur Fütterung von Wiederkäuern.**

(57) Natrium-, Kalium-, Ammonium-, Magnesium- oder Calcium-Salze des Methionins, gegebenenfalls in Form von wäßrigen Lösungen, werden in einer Menge von 0,01 bis 5 Gew.%, bezogen auf die Trockensubstanz der Gesamtfuttermenge, zur Fütterung von Wiederkäuern verwendet.

EP 0 130 281 A1

Degussa Aktiengesellschaft
Weißfrauenstraße 9, 6000 Frankfurt 1

# Verwendung von Salzen des Methionins zur Fütterung von Wiederkäuern

## Beschreibung:

Gegenstand der Erfindung ist die Verwendung von Salzen des Methionins mit der allgemeinen Formel

$$\left[ CH_3 - S - CH_2 - CH_2 - \overset{\displaystyle H}{\underset{\displaystyle NH_2}{\overset{|}{\underset{|}{C}}}} - COO \right]^- X^+$$

in der X ein Äquivalent Natrium, Kalium, Ammonium, Magnesium oder Calcium bedeutet, in einer Menge zwischen 0,01 und 5 Gewichtsprozent, berechnet als Methionin und bezogen auf die Trockensubstanz der Gesamtfuttermenge, zur Fütterung von Wiederkäuern.

Es ist bekannt, Aminosäuren als ernährungsphysiologisch wirksame Zusatzstoffe für Futtermittel und Mischfutter zu verwenden. So bewirkt beispielsweise die Zulage von Methionin zu Rationen, in denen diese Aminosäure in einer für das Tier nicht ausreichenden Menge vorhanden ist, eine Verbesserung der Methioninversorgung und der Eiweißausnutzung. Als Folge dieser Maßnahme werden Zuwachsrate und Futterverwertung des Tieres gesteigert. Diese Erfolge lassen sich jedoch bisher mit freien Aminosäuren nur an monogastrischen Tieren erzielen.

Der Grund dafür ist, daß sich die Ernährungsphysiologie der Wiederkäuer grundsätzlich von der der monogastrischen Tiere unterscheidet. Wiederkäuer besitzen bekanntlich mehrere Mägen. Der erste und weitaus größte Magen, der Pansen, enthält eine eigene Mikroflora aus Bakterien und Protozoen, die alle Nährstoffe des Futters mikrobiell abbaut. Von den Futterproteinen passieren so trotz ihres hochmolekularen und schwerlöslichen Charakters weniger als 30 % der aufgenommenen Eiweißstoffe den Pansen unverändert. Mehr als 70 % werden mikrobiell abgebaut. Die Abbaurate der Futterproteine ist hoch korreliert mit ihrer Löslichkeit im Pansenmedium. Schwerlösliche, denaturierte Proteine werden von den Mikroben relativ langsam angegriffen. Leichtlösliches Eiweiß hingegen wird vollständig abgebaut, wobei über die Stufe der Peptide die freien Aminosäuren entstehen, die sofort weiter mikrobiell zu Ammoniak und kurzkettigen Fettsäuren umgesetzt werden. Freie Aminosäuren, die dem Futter zugesetzt werden, erleiden das gleiche Schicksal. Wegen ihres niedermolekularen Charakters und ihrer vergleichsweise guten Löslichkeit sind sie dem Angriff der Bakterien und Protozoen des Pansens besonders

ausgesetzt (J. Anim. Sci., 14, (1955), 132 - 136). Die Geschwindigkeit der mikrobiellen Umsetzung von Aminosäuren im Pansenmedium ist ebenfalls hoch korreliert mit ihrer Löslichkeit. So wird z.B. das sehr gut lösliche Arginin rasch abgebaut, das nicht ganz so gut lösliche Threonin und die verhältnismäßig schwer löslichen Aminosäuren Methionin und Isoleucin entsprechend langsamer [J. Anim. Sci. 43, 828 (1976) (in der nachfolgenden Tabelle 1 als (1) identifiziert) und Tierphysiologie, Tierernährung und Futtermittelkunde 42, 333 (1979) (in der nachfolgenden Tabelle 1 als (2) identifiziert)].

Tabelle 1: Löslichkeit von Aminosäuren und deren Abbaurate durch Pansenmikroben

| Aminosäure | Löslichkeit (g/100 g $H_2O$, bei 20 $^oC$) | Abbaurate (mMol . l Pansensaft$^{-1}$ . $h^{-1}$) (1) | (2) |
|---|---|---|---|
| Arginin | 14,9 | 0,88 | 0,68 - 0,79 |
| Threonin | 9,0 | 0,50 | 0,39 |
| Methionin | 2,9 | 0,09 | 0,28 - 0,32 |
| Isoleucin | 2,3 | 0,21 | 0,27 - 0,35 |

Eine Qualitätsverbesserung des Futters von Wiederkäuern durch den Zusatz von freien Aminosäuren ist daher nicht zu erwarten (J. Anim. Sci. 9, (1950), 661; 10, (1951), 439 bis 446, 1052; 14, (1955), 132 bis 136; Austr. J. Biol. Sci. 34 (1981), 47 bis 59).

Als Konsequenz wurde vielfach versucht, Aminosäuren durch geeignete Maßnahmen "schwerlöslich" und damit mikrobiell schwerer angreifbar zu machen (vgl. z.B. DE-PS 2 205 210,

DE-PS 2 212 568, DE-PS 3 013 000, DE-PS 1 692 452, US-PS 4 181 709, DE-PS 2 307 836 oder DE-OS 2 536 954). Der Zusatz derartig geschützter Aminosäuren, die von den Bakterien und Protozoen nicht mehr abgebaut, in den an den Pansen anschließenden Abschnitten des Verdauungsapparates aber für das Tier wieder verfügbar werden, zum Futter hat sich als eine wirksame Methode zur Verbesserung der Aminosäuren- und Eiweißversorgung von Wiederkäuern erwiesen.

Überraschenderweise wurde jetzt gefunden, daß die für Wiederkäuer am häufigsten limitierende Aminosäure Methionin sehr vorteilhaft durch die völlig entgegengesetzte Maßnahme, nämlich durch "Leichtlöslichmachen" des Methionins in erheblichem Maße vor mikrobiellem Abbau im Pansen geschützt werden kann. Vorzugsweise eignen sich hierzu als Methioninverbindungen die Natrium-, Kalium-, Ammonium-, Magnesium- und Calciumsalze der Aminosäure, die sowohl in Form von wässrigen Lösungen in das Futter eingemischt als auch in Pulverform Anwendung finden können - mit Ausnahme des Ammoniumsalzes des Methionins, das nur in wässriger Lösung existiert.

Die erfindungsgemäß zu verwendenden Salze des Methionins sind dadurch gekennzeichnet, daß sie eine zum Teil wesentlich höhere Löslichkeit besitzen als die freie Aminosäure Methionin in ihrer DL-, L- oder D-Form. So lassen sich beispielsweise bei 20 °C in Wasser ohne Schwierigkeiten 50 gewichtsprozentige wässrige Lösungen von Natrium-DL-methioninat herstellen. Die Löslichkeit von DL-Methionin in Wasser bei 20 °C liegt vergleichsweise unter 3 Gewichtsprozent. Die genannten Salze des Methionins können vorteilhaft durch Umsetzung von Methionin mit den entsprechenden Alkali- und Erdalkalihydroxiden oder -oxiden in wässriger Lösung hergestellt werden. Auch die direkte Hydrolyse von 5 [(ß-Methylmercapto)-ethyl] -hydantoin durch entsprechende Alkali- und/oder Erdalkalihydroxide ist zur Herstellung geeignet. Sie ist insbesondere dann von Vorteil, wenn als Ziel der Gewinnung der Methioninverbindung eine wässrige Lösung des Produktes angestrebt wird.

Die erfindungsgemäße Verwendung der Salze des Methionins im Futter erfolgt im allgemeinen durch Zumischen zu üblichen Futtermitteln und Mischfuttern für Wiederkäuer. Es können aber auch spezielle Vormischungen mit Vitaminen und Mineralstoffen, Futterharnstoff oder Energieträgern verwendet werden. Die Menge der zu verabreichenden Salze des Methionins soll vorzugsweise so bemessen sein, daß innerhalb der Gesamtfuttermenge der zugeführte Methioninanteil, bezogen auf Trockensubstanz, 0,05 bis 1 Gewichtsprozent beträgt.

Methionin ist für Wiederkäuer ebenso wie für viele monogastrische Tierarten erstlimitierende Aminosäure. Der spezifische Grund für den Methioninmangel der Wiederkäuer ist, daß ihre Futterrationen weitgehend unabhängig von der ursprünglichen Zusammensetzung im Pansen mikrobiell zum größten Teil ab- bzw. umgebaut werden. Auf der Proteinseite der Futter ist die Aminosäurenversorgung des Magen-Darmtraktes und damit die des Gesamtorganismus im wesentlichen gebunden an die genetisch festgelegte Aminosäurenzusammensetzung des Mikrobenproteins, das bekanntlich eine Methioninlücke hat. Da andererseits bekannt ist, daß Wiederkäuer sowohl für Milchleistung als auch für Wachstum und Woll- bzw. Fellproduktion einen hohen Bedarf an schwefelhaltigen Aminosäuren, beispielsweise Methionin, haben, läßt sich die Leistung der Wiederkäuer wie die der monogastrischen Tiere durch Bereitstellung zusätzlicher Mengen der erstlimitierenden Aminosäure Methionin am Ort der Synthese der körpereigenen Proteine erhöhen (vgl. z.B. Fed. Proc. Am. Soc. Exp. Biol. 29, 1970, 44 bis 50; Z. Tierphysiologie, Tierernährung und Futtermittelkunde 41 (4), 1979, 202 bis 217; Austr. J. Biol. Sci. 16, 1, 1963, 218 bis 230).

Die Erfindung soll durch die nachfolgend beschriebenen Beispiele näher erläutert werden:

Beispiel 1:

Die Wirksamkeit von Natrium-DL-methioninat zur Erhöhung der im Stoffwechsel verfügbaren Methioninmenge wurde in Fütterungsversuchen an 4 Milchkühen geprüft. Den Tieren wurde zu einer dem Bedarf entsprechenden Ration aus Grund- und Kraftfutter täglich eine 1 kg-Vormischung mit 57 g Natrium-DL-methioninat verabreicht. Die Vormischung hatte folgende Zusammensetzung (Gewichtsprozente):

| | |
|---|---|
| Getreideschrote | 43 % |
| Maiskleberfutter | 35 % |
| Melasse | 5 % |
| Kokosschrot | 8 % |
| Mineralstoffe | 3 % |
| Natrium-DL-methioninat* | 5,7 % |
| Vitaminvormischung | 0,3 % |

* Bei der zum Vergleich vorgenommenen Verabreichung von DL-Methionin wurde die freiwerdende Differenzmenge in Form von Getreideschrot gegeben; in der Periode ohne Methioninzusatz wurde der Zusatz komplett durch Getreideschrot ersetzt.

Nach einer Adaptationszeit von 3 Tagen wurden an 4 aufeinanderfolgenden Tagen jeweils zum Zeitpunkt der Fütterung, sowie 1, 2 und 3 Stunden danach Blutproben aus der Vena Jugularis entnommen. Diese Proben wurden auf freies Methionin im Plasma untersucht. Zum Vergleich wurde jeweils eine Kontrollperiode mit DL-Methionin als Zulage und eine Kontrollperiode ohne zusätzliche Methioninzulage herangezogen.

Die Ergebnisse sind in der folgenden Tabelle (2) zusammengefaßt:

Tabelle 2: Plasmamethioningehalt (mg/100 ml) von Kühen nach Verabreichung von Methioninzulagen (in Form von Natrium-DL-methioninat oder DL-Methionin

| Zeitpunkt der Blutentnahme nach Fütterung (Stunden) | 0 | 1 | 2 | 3 | $\bar{x}$ (1 - 3) |
|---|---|---|---|---|---|
| **Tier 1** | | | | | |
| Kontrollperiode | 0,288 | 0,258 | 0,238 | 0,269 | 0,255 |
| Versuchsperiode mit Natrium-DL-methioninat | 0,355 | 0,366 | 0,408 | 0,466 | 0,413 |
| Versuchsperiode mit DL-Methionin | 0,316 | 0,307 | 0,431 | 0,412 | 0,383 |
| **Tier 2** | | | | | |
| Kontrollperiode | 0,346 | 0,298 | 0,282 | 0,230 | 0,270 |
| Versuchsperiode mit Natrium-DL-methioninat | 0,319 | 0,554 | 0,852 | 1,067 | 0,824 |
| Versuchsperiode mit DL-Methionin | 0,319 | 0,402 | 0,653 | 0,849 | 0,635 |
| **Tier 3** | | | | | |
| Kontrollperiode | 0,368 | 0,282 | 0,236 | 0,220 | 0,246 |
| Versuchsperiode mit Natrium-DL-methioninat | 0,348 | 0,372 | 0,688 | 0,735 | 0,598 |
| Versuchsperiode mit DL-Methionin | 0,344 | 0,312 | 0,426 | 0,373 | 0,370 |
| **Tier 4** | | | | | |
| Kontrollperiode | 0,358 | 0,311 | 0,288 | 0,234 | 0,278 |
| Versuchsperiode mit Natrium-DL-methioninat | 0,360 | 0,421 | 0,847 | 0,801 | 0,640 |
| Versuchsperiode mit DL-Methionin | 0,370 | 0,508 | 0,432 | 0,560 | 0,500 |
| **Tier 1 bis 4 (Durchschnitt)** | | | | | |
| Kontrollperiode | 0,340 | 0,288 | 0,261 | 0,238 | 0,262 |
| Versuchsperiode mit Natrium-DL-methioninat | 0,346 | 0,428 | 0,699 | 0,767 | 0,631 |
| Versuchsperiode mit DL-Methionin | 0,387 | 0,382 | 0,486 | 0,549 | 0,472 |

Beispiel 2:

In einem Kälbermastversuch mit 12 frühabgesetzten Tieren im Gewichtsabschnitt von 80 bis 120 kg wurde die Wirksamkeit von Kalium-DL-methioninat geprüft. An Gruppen von jeweils 4 Tieren wurden Kraftfuttermischungen gefüttert, die keine Methioninergänzung, 10 g DL-Methionin und 36,1 g einer 35 gewichtsprozentigen wässrigen Kaliummethioninatlösung enthielten. Den Kälbern wurde zusätzlich zum Kraftfutter eine standardisierte Menge Grundfutter (Heu) verabreicht. Die Kraftfuttermenge wurde limitiert, um in allen Gruppen eine gleiche Futteraufnahme zu erhalten. Die Ergebnisse des Versuches zeigt die folgende Tabelle (3):

Tabelle 3: 42-Tage-Fütterungsversuch mit frühabgesetzten Kälbern (Rasse rotbunt)

| Gruppe | I Kontrollgruppe ohne Zulage | II Versuchsgruppe mit DL-Methionin | III Versuchsgruppe mit Natrium-DL-methioninat |
|---|---|---|---|
| Tierzahl | 4 | 4 | 4 |
| Futteraufnahme (kg) (Kraftfutter) | 2,2 | 2,2 | 2,2 |
| Anfangsgewicht (kg) | 80,1 | 80,8 | 79,9 |
| Endgewicht (kg) | 117,3 | 119,2 | 120,4 |
| Durchschnittliche tägliche Zunahme (g) | 886 | 914 | 967 |

Beispiel 3:

8 Milchkühe in der 3. Laktation wurden in 2 Gruppen à 4 Tiere aufgeteilt. Die Versuchsgruppe erhielt täglich 34,5 g Methionin in Form von Calcium-DL-methioninat, entsprechend 40 g Calciummethioninat mit 98 % Reinheit, das in 960 g Kraftfutter eingemischt war.

Die Grundfutterration der Kühe bestand aus Maissilage, Grassilage und Heu. Kraftfutter wurde nach Leistung dosiert verabreicht.

Die Kontrollgruppe wurde in gleicher Weise gefüttert, mit der Ausnahme, daß anstelle des Methioninsupplements Kraftfutter verabreicht wurde. Nach einer Versuchsperiode von 4 Wochen wurden Kontrollgruppe und Versuchsgruppe getauscht und die gleichen Parameter Milchmenge, Milchfett- und Milcheiweißgehalt ermittelt. Die Ergebnisse zeigt Tabelle (4):

Tabelle 4: Wirkung von Calcium-DL-methioninat als Zusatz im Milchleistungsfutter

| Tier-Nr. | 1. Periode | | | 2. Periode | | |
|---|---|---|---|---|---|---|
| | Milch (kg) | Fett (%) | Eiweiß (%) | Milch (kg) | Fett (%) | Eiweiß (%) |
| | (mit Ca-DL-methioninat) | | | (ohne Ca-DL-methioninat) | | |
| 1 | 15,5 | 3,6 | 3,3 | 12,9 | 4,2 | 3,3 |
| 2 | 19,7 | 5,3 | 3,4 | 19,8 | 5,0 | 3,4 |
| 3 | 28,2 | 3,8 | 2,9 | 27,6 | 3,6 | 3,1 |
| 4 | 26,5 | 5,6 | 3,6 | 25,1 | 4,0 | 2,8 |
| Ø | 22,5 | 4,6 | 3,3 | 21,4 | 4,2 | 3,2 |
| | (ohne Ca-DL-methioninat) | | | (mit Ca-DL-methioninat) | | |
| 5 | 22,7 | 4,4 | 3,3 | 23,2 | 4,0 | 3,6 |
| 6 | 19,0 | 3,3 | 3,2 | 20,7 | 4,5 | 3,3 |
| 7 | 20,9 | 5,0 | 3,5 | 22,8 | 4,8 | 3,2 |
| 8 | 24,3 | 3,7 | 2,8 | 27,0 | 4,0 | 3,3 |
| Ø | 21,7 | 4,1 | 3,2 | 23,4 | 4,3 | 3,4 |

Die Ergebnisse der Beispiele 2 und 3 belegen, daß die erfindungsgemäße Verwendung von Salzen des Methionins, sowohl in gelöster als auch in fester Form, geeignet ist, die Methioninversorgung von Wiederkäuern im Hinblick auf Gewichtszunahme und Milchleistung zu verbessern.

0130281
83 173 AM

D e g u s s a Aktiengesellschaft
Weißfrauenstraße 9, 6000 Frankfurt 1

# Verwendung von Salzen des Methionins zur Fütterung von Wiederkäuern

## Patentanspruch:

Verwendung von Salzen oder wässrigen Lösungen von Salzen des Methionins mit der allgemeinen Formel

$$\left[ CH_3 - S - CH_2 - CH_2 - \underset{NH_2}{\overset{H}{\underset{|}{\overset{|}{C}}}} - COO \right]^- X^+$$

in der X ein Äquivalent Natrium, Kalium, Ammonium, Magnesium oder Calcium bedeutet, in einer Menge zwischen 0,01 und 5 Gewichtsprozent, berechnet als Methionin und bezogen auf die Trockensubstanz der Gesamtfuttermenge, zur Fütterung von Wiederkäuern.

Europäisches Patentamt

# EUROPÄISCHER RECHERCHENBERICHT

0130281 Nummer der Anmeldung

EP 84 10 3508

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| Y | DE-A-2 704 746 (INTERCHEMIE) * Patentansprüche 1,2 * | 1 | A 23 K 1/18<br>A 23 K 1/16 |
| | --- | | |
| Y | CH-A- 603 069 (INTERCHEMIE) * Patentansprüche I,II; Unteransprüche 2,3 * | 1 | |
| | --- | | |
| Y | EP-A-0 058 771 (DEGUSSA) * Patentanspruch * | 1 | |
| | --- | | |
| A | GB-A-1 166 543 (SUMITOMO CHEMICAL COMP. LTD.) | | |
| | ----- | | RECHERCHIERTE SACHGEBIETE (Int. Cl. 3) |
| | | | A 23 K |

Der vorliegende Recherchenbericht wurde für alle Patentansprüche erstellt.

| Recherchenort | Abschlußdatum der Recherche | Prüfer |
|---|---|---|
| DEN HAAG | 05-10-1984 | DEKEIREL M.J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82